# Europäisches Patentamt

# European Patent Office

# Office européen des brevets

(19)

(11) Veröffentlichungsnummer: **0 124 714**
**B1**

(12) **EUROPÄISCHE PATENTSCHRIFT**

(45) Veröffentlichungstag der Patentschrift:
15.10.86

(21) Anmeldenummer: 84102506.7

(22) Anmeldetag: 08.03.84

(51) Int. Cl.⁴: **A 61 B 6/14, G 03 B 42/02**

(54) Röntgendiagnostikeinrichtung für Zahnaufnahmen.

(30) Priorität: 07.04.83 DE 3312520

(43) Veröffentlichungstag der Anmeldung:
14.11.84 Patentblatt 84/46

(45) Bekanntmachung des Hinweises auf die Patenterteilung:
15.10.86 Patentblatt 86/42

(84) Benannte Vertragsstaaten:
DE FR GB IT SE

(56) Entgegenhaltungen:
DE-A-2 740 356
FR-A-962 963
GB-A-976 477
US-A-3 092 721
US-A-3 745 344
US-A-4 012 638
US-A-4 048 506
US-A-4 109 156
US-A-4 166 220

(73) Patentinhaber: Siemens Aktiengesellschaft Berlin und München, Wittelsbacherplatz 2, D-8000 München 2 (DE)

(72) Erfinder: Jeck, Günter, Am Bannelsbach 13a, D-6149 Rimbach (DE)
Erfinder: Scheller, Karl, Am Wetzelsbach 22, D-6941 Gorxheimertal (DE)
Erfinder: Schmitt, Ernst, Altengassweg 42, D-6140 Bensheim (DE)
Erfinder: Schubert, Gustav, Hügelstrasse 55, D-6143 Lorsch (DE)

**Beschreibung**

Die Erfindung bezieht sich auf eine Röntgendiagnostikeinrichtung für Zahnaufnahmen, die mit Hilfe einer extern eines Patientenmundes plazierbaren Röntgenstrahlenquelle und eines mittels eines Filmhalters im Patientenmund positionierbaren Filmes gemacht werden und bei der der Filmhalter so beschaffen und mittels einer Tragstange an einem röhrenförmigen Tubus so befestigt ist, daß der Film rechtwinkelig zur Tubusachse verläuft.

Bei einer bekannten solchen Röntgendiagnostikeinrichtung (US-PS 41 50 296) enthält der Tubus, der einen beliebigen Querschnitt haben kann, eine Umkreisnut mit einem Ring, der in verschiedenen Stellungen gedreht und fixiert werden kann. Der Ring wiederum enthält eine Halterung für eine Stange, an derem freien Ende mittels eines Haltegliedes der zu belichtende Film gehaltert werden kann. Die Halterung ist so beschaffen, daß die Tragstange des Filmhalters um ihre eigene Längsachse gedreht und entlang dieser verschoben und in der Lage der Verschiebungsrichtung fixiert werden kann.

Durch diese Anordnung kann der Röntgenfilm sich in einer Ebene rechtwinkelig zur Achse des Tubus und damit zur Röntgenröhre um die Achse der Tragstange und um die Achse der Röhre bewegen. Darüber hinaus kann der Film zum Tubus hin bzw. vom Tubus weg bewegt werden.

Das Positionieren des Filmes im Patientenmund erfolgt bei dieser bekannten Einrichtung so, daß der Film zunächst in die gewollte Lage in Relation zu-m Tubusende gebracht wird und danach in den Patientenmund eingeführt wird.

Das Einführen, Adaptieren und Positionieren des Filmes bereitet bei der bekannten Röntgendiagnostikeinrichtung insofern gewisse Probleme, als stets die gesamte Röntgendiagnostikeinrichtung und damit eine relativ große Masse bewegt werden muß. Trotz der kardanischen Aufhängung der Einrichtung ist jedoch kein feinfühliges Verstellen möglich; letztlich auch wegen der relativ schlechten Sichtverhältnisse. Darüber hinaus besteht durch die in fester Zuordnung von Filmhalter und Tubus befindliche Verbindung bei einer Nachjustierung eine gewisse Verletzungsgefahr bzw. die Gefahr, daß beim Patienten ein Brechreiz hervorgerufen wird.

Aufgabe der vorliegenden Erfindung ist es, eine Röntgendiagnostikeinrichtung der eingangs genannten Gattung dahingehend zu verbessern, daß das Einführen, Adaptieren und Positionieren von Film und Tubus erreichtet wird und die obengenannten Nachteile vermieden werden.

Zur Lösung der gestellten Aufgabe werden die im Anspruch 1 angegebenen Maßnahmen vorgeschlagen. Vorteilhafte Weiterbildungen und Ausgestaltungen der Erfindung sind in den Unteransprüchen enthalten.

Ein Ausführungsbeispiel der Erfindung wird nachfolgend anhand der Zeichnung näher erläutert.

Es zeigen:

Fig. 1 eine zahnärztliche Röntgendiagnostikeinrichtung gemäß der Erfindung in einer schaubildlichen Darstellung,

Fig. 2 einen Teil der Anordnung nach Fig. i im Längsschnitt entlang der Linie II-II,

Fig. 3 bis 7 Einzelheiten des Filmhalters.

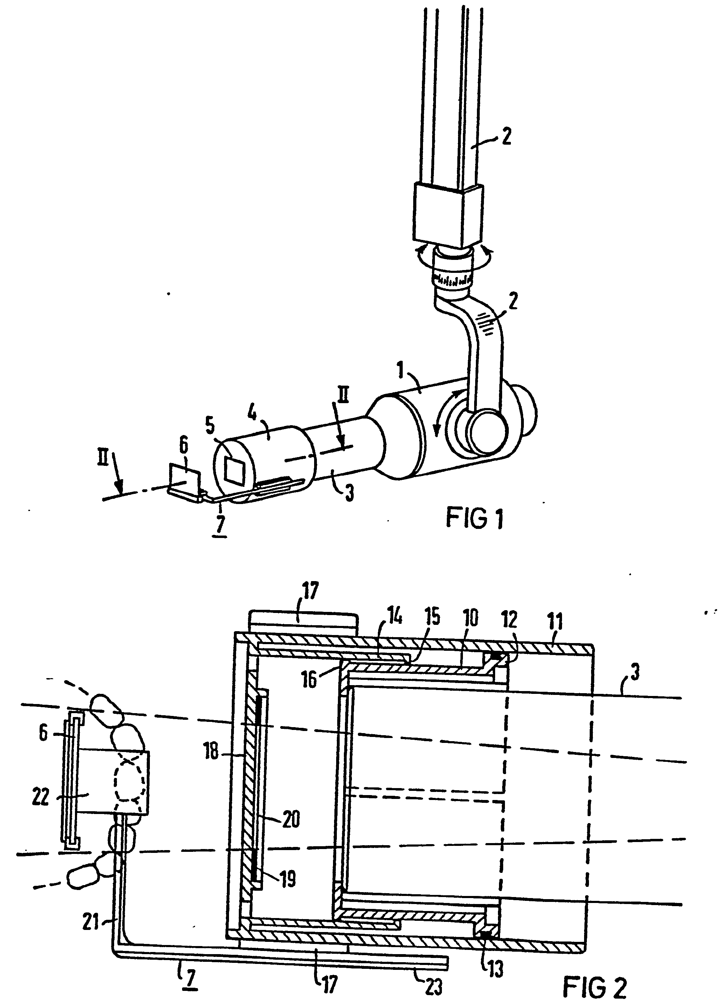

Die Fig. 1 zeigt in einer schaubildlichen Darstellung eine zahnärztliche Röntgendiagnostikeinrichtung der erfindungsgemäßen Art. Ein Gehäuse 1, welches mittels nicht näher erläuterter Tragarme 2 kardanisch aufgehängt ist, enthält eine nicht dargestellte Röntgenstrahlenquelle. Das von der Röntgenstrahlenquelle erzeugte Strahlenbündel wird durch einen langgestreckten Tubus 3 geleitet, der eine später noch näher erläuterte Zieleinrichtung 4 mit einer dem Filmformat entsprechenden Markierung 5 enthält. An der Zieleinrichtung 4 ist ein den Röntgenfilm 6 aufnehmender Filmhalter 7 angeordnet.

Die Fig. 2 zeigt eine vorteilhafte Ausführungsform der auf den Tubus 3 auf setzbaren Zieleinrichtung 4. Die Zieleinrichtung 4 besteht im wesentlichen aus zwei teleskopartig ineinandergreifenden Hülsen, einer Adapterhülse 10 mit einem dem Außendurchmesser des Tubus 3 angepaßten Innendurchmesser und einer konzentrisch dazu angeordneten Verstellhülse 11. Die Adapterhülse 10 ist im Durchmesser abgestuft und bildet. einen Bund 12 mit einem in einer Ringnut eingelegten O-Ring 13; die Verstellhülse. 11 weist einen zur Führung der Adapterhülse 10 dienenden hülsenförmigen Ansatz 14 auf.

Vorsprünge 15 16, einerseits am hülsenförmigen Ansatz 14 und andererseits an der Adapterhülse 10, sorgen einerseits für eine reibungsarme Führung und andererseits für eine axiale Begrenzung der Verstellmöglichkeit für die Verstellhülse 11.

Die Verstellhülse 11 weist am Umfang zwei später noch näher erläuterte Haltespangen zur Aufnahme des Filmhalters 7 auf.

Beide Hülsen bestehen aus Kunststoff, sind also röntgenstrahlendurchlässig. Die Verstellhülse 11 ist frontseitig geschlossen; hinter der Stirnwand 18 ist eine Blende 19 aus für Röntgenstrahlen undurchlässigem Material, z.B. Blei, angeordnet. Diese Blende enthält ein dem Filmformat (30 x 40 mm) entsprechend großes Fenster 20; in diesem Bereich ist also die Strahlung durchlässig. Um von außen die Lage des Fensters erkennen zu können, ist an der Stirnwand 18 die in Fig. 1 mit 5 bezeichnete Markierung vorgesehen.

Aus dem beschriebenen Aufbau der Zieleinrichtung geht hervor, daß nach Aufsetzen der Position 4 auf den Tubus 3 die Verstellhülse gegenüber dem Tubus und damit der 25 Röntgenstrahlenquelle sowohl verdreht als auch in axialer Richtung verschoben werden kann. Dadurch ist ein besonders feinfühliges Anpassen an den Patientenmund möglich, ohne daß hierzu das relativ schwere und damit schwerfällig zu verstellende Gehäuse einschließlich der Tragarme bewegt zu werden braucht.

Nachfolgend wird die Filmhalterung näher beschrieben.

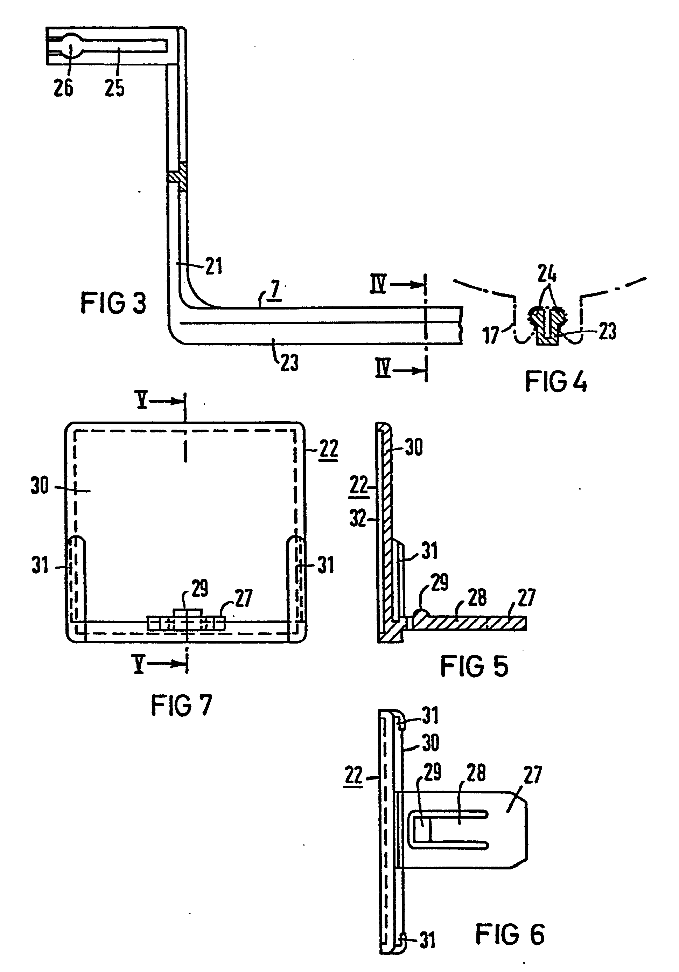

Der Filmhalter 7 besteht aus zwei Teilen, einer mehrfach abgewinkelten Tragstange 21 (Fig. 3) und einem Halteglied 22, an dem der Film befestigt werden kann. Die Teile 21 und 22 sind durch eine noch näher erläuterte Rastvorrichtung miteinander verbindbar. Der mit 23 bezeichnete eine Schenkel der Filmhaltestange 21 hat den in Fig. 4 gezeigten Querschnitt; ein U-Profil mit seitlichen Spannbacken 24, die beim Aufsetzen des Filmhalters auf eines der mit 17 bezeichneten Haltespangen, die ebenfalls V- oder U-förmig geformt und einseitig offen sind, in Eingriff kommen. Durch diese Art der Halterung ist einerseits eine Längsverschiebbarkeit des Filmhalters in bzw. parallel zur Zentrumsachse des Tubus bzw. der Zieleinrichtung 4 gegeben, andererseits kann bei einer seitlichen Auslenkung des Filmhalters dieser aus der Haltespange 17 ausrücken. Durch diese Nachgiebigkeit wird die Gefahr einer Verletzung der Schleimhäute im Patientenmund weitgehend ausgeschaltet.

Das das Halteglied 22 aufnehmende Ende der Filmhaltestange 21 ist mit einem Schlitz 25 sowie einer balligen Ausnehmung 26 versehen; dieser Konstruktion entsprechend enthält das Filmhalteglied 22 eine in den Schlitz 25 einführbare Lasche 27 mit einer federnden Zunge 28, die eine Anformung 29 aufweist, die in die Ausnehmung 26 der Filmhaltestange 21 eingreift.

An die Lasche 27 angeformt ist eine Rückwand 30, die seitlich mit Führungs- und Haltemitteln 31 für den Film versehen ist. Die Rückwand 30 dient einerseits, um den aufzunehmenden Film während der Aufnahme plan zu haltern, andererseits um eine mit 32 bezeichnete Platte aus röntgenstrahlenundurchlässigem Material, z.B. Blei, aufzunehmen. Durch diese zusätzlich zu der bereits beschriebenen Blende 19 (Fig. 2) vorgesehenen weiteren, röntgenstrahlenabsorbierenden Maßnahme läßt sich die Strahlenbelastung, wie anhand des in Fig. 1 eingezeichneten Strahlenverlauf s skizziert, noch weiter reduzieren.

Nachdem das Filmhalteglied lösbar mit der Baltestange 21 verbunden ist, kann, je nach aufzunehmendem Film, (Hoch- oder Querformat, Spezialfilm für Kindergebiß 10 od. dgl.) ein an den Film entsprechend angepaßtes Filmhalteglied auf ein und dieselbe Filmhaltestange aufgesetzt werden.

Zur Vorbereitung einer Aufnahme wird zunächst das gesamte Röntgengerät grob auf den Patientenmund bzw. auf das auf zunehmende Objekt eingestellt. Danach wird dem Patienten der Filmhalter mit dem eingelegten Film in den Mund gegeben. Eine Anpassung und Positionierung des Tubus an den Filmhalter kann nunmehr sehr leicht durch den Zoom-Effekt der Zieleinrichtung 4 sowie durch die freie Drehbarkeit der Verstellhülse 11 gegenüber der Adapterhülse 10 feinfühlig eingestellt werden, ohne daß hierzu die gesamte Diagnostikeinrichtung verstellt zu werden braucht.

## Patentansprüche

1. Röntgendiagnostikeinrichtung für Zahnaufnahmen, die mit Hilfe einer extern eines Patientenmundes plazierbaren Röntgenstrahlenquelle und eines mittels eines Filmhalters (7) im Patientenmund positionierbaren Filmes (6) gemacht werden und bei der der Filmhalter so beschaffen und mittels einer Tragstange (21) an einem röhrenförmigen das Strahlenbündel leitenden Tubus (3) so befestigt ist, daß der Film rechtwinkelig zur Tubusachse verläuft, dadurch gekennzeichnet, daß am Tubus (3) eine Zieleinrichtung (4) mit einer gegenüber dem Tubus drehbaren und in Achsrichtung verstellbaren Rülse (11) vorhanden ist, die am Umfang wenigstens eine einseitig offene Halterung (17) für den Filmhalter (7) enthält, die so beschaffen ist, daß einerseits ein axiales Einführen und, bezogen auf die Tubusachse, achsparalleles Verschieben des Filmhalters und andererseits ein Ausklinken des Filmhalters aus der Halterung bei einer seitlichen Krafteinwirkung gegeben ist.

2. Röntgendiagnostikeinrichtung nach Anspruch 1, dadurch gekennzeichnet, daß die Zieleinrichtung (4) aus zwei konzentrisch zueinander angeordneten gegeneinander verdrehbaren und teleskopartig in axialer Richtung gegeneinander verstellbaren Hülsen (10, 11) besteht, von denen die Innenhülse (10) am Tubus ortsfest angeordnet ist und die Außenhülse (11) die Halterung (17) für den Filmhalter (7) trägt.

3. Röntgendiagnostikeinrichtung nach Anspruch 2, dadurch gekennzeichnet, daß die Innenhülse (10) in ihrem Außendurchmesser abgestuft ist und so einen Bund (12) bildet, der eine Nut mit einem darin eingelegten O-Ring (13) enthält, und daß die Außenhülse (11) einen konzentrisch angeordneten hülsenförmigen Ansatz (14) enthält, der mit einem radial gerichteten Vorsprung (15) auf der im Durchmesser abgestuften Fläche der Innenhülse aufliegt und so zusammen mit dem O-Ring eine Axialführung bildet.

4. Röntgendiagnostikeinrichtung nach Anspruch 3, dadurch gekennzeichnet, daß der Vorsprung (15) am Ansatz (14) der Außenhülse (11) und ein weiterer Vorsprung (16) am freien Ende der Innenhülse (10) Anschlagmittel für eine axiale Begrenzung der Verstellbarkeit der Außenhülse (11) bilden.

5. Röntgendiagnostikeinrichtung nach einem der Ansprüche 2 bis 4, dadurch gekennzeichnet, daß die Außenhülse (11) stirnseitig durch eine den Strahlenaustritt auf das Filmformat begrenzende Blende (19) aus strahlenundurchlässigem Material begrenzt ist und die Stirnwand (18) eine von außen sichtbare, etwa dem Filmformat entsprechende rechteckige Markierung (5) enthält.

6. Röntgendiagnostikeinrichtung nach einem der Ansprüche 2 bis 5, dadurch gekennzeichnet, daß die Zieleinrichtung (4) lösbar am Tubus (3) angeordnet ist, wozu die Innenhülse (10) vorzugsweise als Steckhülse ausgebildet ist und mit dem Tubus (3) formschlüssig verbindbar ist.

7. Röntgendiagnostikeinrichtung nach einem der Ansprüche 2 bis 6, dadurch gekennzeichnetz, daß an der Außenhülse (11) wenigstens zwei am Umfang in ejner 90°-Teilung angeordnete Halterungen (17) für den Filmhalter befestigt sind.

8. Röntgendiagnostikeinrichtung nach einem der Ansprüche 2 bis 7, dadurch gekennzeichnet, daß der an der Außenhülse (11) befestigbare Filmhalter eine Anlagefläche für den Film (6) aufweist und eine die Anlagefläche bildende Rückwand (30) aus strahlenundurchlässigem Material besteht oder eine aus solchem Material bestehende Platte (32) enthält.

9. Röntgendiagnostikeinrichtung nach Anspruch 8, dadurch gekennzeichnet, daß die Platte (32) in einem L-förmig gestalteten Halteglied (22) integriert ist, dessen einer Schenkel die Anlagefläche für den Film (6) bildet und dessen anderer Schenkel zur lösbaren Befestigung an der Tragstange (21) ausgebildet ist und daß das den Film tragende Halteglied (22) des Filmhalters (7) an der am Tubus befestigbaren Tragstange (2i) abnehmbar gehaltert ist.

10. Röntgendiagnostikeinrichtung nach Anspruch 9, dadurch gekennzeichnet, daß die Tragstange (21) und ihre Halterung (17) am Tubus (3) im Querschnitt U- oder V-förmig ausgebildet sind und so seitlich federnde Spannbacken (24) bilden, die mit in dazu passenden Führungs- und Haltespangen (25) am Tubus in und außer Eingriff bringbar sind.

## Claims

1. X-ray diagnostic apparatus for dental photographs taken with the aid of an X-ray radiation source placed outside the patient's mouth and a film (6) positioned inside the patient's mouth in a film holder (7) held by a supporting rod (21) carrying a guide tube (3) which guides the X-ray beam in such a manner that the film runs at right angles to the tube axis, characterised in that on the guide tube (3) there is arranged a target device (4) having a sleeve (11) rotatable in relation to the guide tube and adjustable in the axial direction and which on the periphery comprises at least one unilaterally open holder (17) for the film holder (7), which holder (17) is such that axial insertion is provided for, and displacement of the film holder parallel to the tube axis, and that the film holder can be disengaged from the holder by the influence of a lateral force.

2. X-ray diagnostic apparatus as claimed in Claim 1, characterised in that the target device (4) consists of two concentrically arranged and mutually rotatable sleeves (10,11) which are telescopically adjustable in the axial direction and the inner sleeve (10) of which is stationary on the guide tube and the outer sleeve (11) carries the holder (17) for the film holder (7).

3. X-ray diagnostic apparatus as claimed in Claim 2, characterised in that the outer diameter of the inner sleeve (10) is stepped to form a flange (12) which comprises a groove having an O-ring (13) embedded therein, and that the outer sleeve (11) comprises a concentrically arranged sleeve-shaped attachment (14) with a radially directed projection (15) that rests on the surface of the inner sleeve, whose diameter is stepped, and forms an axial guide together with the O-ring.

4. X-ray diagnostic apparatus as claimed in Claim 3, characterised in that the projection (15) on the attachment (14) of the outer sleeve (11) and a further projection (16) at the free end of the inner sleeve (10) form stop m eans for an axial r estriction of the adjustability of the outer sleeve (11).

5. X-ray diagnostic apparatus as claimed in one of Claims 2 to 4, characterised in that at the end face the outer sleeve (11) is bounded by a diaphragm (19) which consists of a material impervious to radiation and which restricts the radiation outlet onto the film format, and the end face (18) comprises a rectangular marker (5) which is visible from the outside and approximately corresponds to the film format.

6. X-ray diagnostic apparatus as claimed in one of Claims 2 to 5, characterised in that the target device (4) is detachably arranged on the guide tube (3), for which purpose the inner sleeve (10) is preferably designed as a plug socket and can be connected to the guide tube (3) in a form-locking manner.

7. X-ray diagnostic a pparatus as claimed in one of Claims 2 to 6, characterised in that at least two holders (17) for the film holder are arranged secured to the outer sleeve (11) on the periphery in a 90° spacing.

8. X-ray d iagnostic apparatus as claimed in one of Claims 2 to 7, characterised in that the film holder which can be secured to the outer sleeve (11) possesses a contact surface for the film (6) and a rear wall (30) which forms the contact surface and consists of a material impermeable to radiation, or comprises a plate (32) which consists of such material.

9. X-ray diagnostic apparatus as claimed in Claim 8, characterised in that the plate (32) is integrated in an L-shaped holding element (22), whose one flank forms the contact surface for the film (6) and whose other flank is designed for detachable fastening on the supporting rod (21), and that the holding element (22) of the film holder (7), which supports the film, is removably supported on the supporting rod (21) which can be secured to the guide tube.

10. X-ray diagnostic apparatus as claimed in Claim 9, <u>characterised in that</u> the supporting rod (21) and its holder (17) on the guide tube (3) have U-shaped or V-shaped crosssection and thus form laterally resilient clamping jaws (24) which can be engaged and disengaged on the guide tube by means of matching guiding and holding clips (25).

## Revendications

1. Installation de radiodiagnostic pour des radiographies dentaires, qui peuvent être réalisées à l'aide d'une source de rayonnement X pouvant être disposée à l'extérieur de la bouche d'un patient, et à l'aide d'un film (6) pouvant être positionné dans la bouche du patient au moyen d'un porte-film (7), et dans lequel le porte-film est agencé et est fixé au moyen d'une barre de support (21) sur un localisateur de forme tubulaire (3) guidant le faisceau de rauonnement, de telle sorte que le film se déplace perpendiculairement à l'axe du localisateur, caractérisé par le fait que sur le localisateur (3) se trouve monté un sélecteur (4) comportant un manchon (11) pouvant être entraîné en rotation par rapport au localisateur et déplaçable suivant la direction axiale et qui continet sur son pourtour, pour le portefilm (7), au moins un support (17) ouvert d'un côté et qui est agencé de manière à permettre d'une part une introduction axiale et un déplacement, parallèle à l'axe du localisateur, du porte-film et d'autre part un dégagement du porte-film hors du support sous l'action d'une force latérale.

2. Installation de radiodiagnostic selon la revendication 1, caractérisée en ce que le sélecteur (4) est constitué par deux manchons (10, 11) concentriques l'un à l'autre, pouvant tourner l'un par rapport à l'autre et déplaçables mutuellement de façon télescopique suivant la direction axiale et parmi lesquels le manchon intérieur (10) est monté fixe sur le localisateur et le manchon extérieur (11) porte le support (17) pour le porte-film (7).

3. Installation de radiodiagnostic suivant la revendication 2, caractérisée par le fait que le diamètre extérieur du manchon intérieur (10) est étagé et que ce manchon possède un collet (12) qui comporte une gorge dans laquelle est insérée un joint torique (13) et que le manchon extérieur (11) contient un embout saillant concentrique en forme de manchon (14), qui est en appui par un organe saillant radial (15) sur la surface de diamètre étagé du manchon intérieur et forne ainsi, avec le joint torique, un système de guidage axial.

4. Installation de radiodiagnostic suivant la revendication 3, caractérisée par le fait que l'organe saillant (15) situé sur l'embout saillant (14) du manchon extérieur (11) et un autre organe saillant (16) prévu sur l'extrémité libre du nanchon intérieur (10) forment des moyens de butée pour une limitation axiale de la capacité de déplacement du manchon extérieur (11).

5. Installation de radiodiagnostic suivant l'une des revendications 2 à 4, caractérisée par le fait que le manchon extérieur (11) est limité, sur sa face frontale, par un diaphragme (19) qui limite la sortie du rayonnement sur le format du film et est constitué en un matériau opaque au rayonnement et que la paroi frontale (18) contient un repère rectangulaire (5) visible de l'extérieur et correspondant approximativement au format du film.

6. Installation de radiodiagnostic suivant l'une des revendications 2 à 5, caractérisé par le fait que le sélecteur (4) est monté de façon détachable sur le localisateur (7), ce qui a pour effet que le manchon intérieur (10) est réalisé de façon appropriée sous la forme d'un manchon enfichable et peut être relié selon une liaison par formes complémentaires au localisateur (3).

7. Installation de radiodiagnostic suivant l'une des revendications 2 à 6, caractérisé par le fait qu'au moins deux supports (17), disposés sur la périphérie selon un pas de répartition de 98°, pour le porte-film sont fixés sur le manchon extérieur (11).

8. Installation de radiodiagnostic suivant l'une des revendications 2 à 7, caractérisé par le fait que le porte-film, qui peut être fixé sur le manchon extérieur (11), comporte une surface d'application pour le film (6) et qu'une paroi arrière (30) constituant la surface d'application est constituée en un matériau opaque au rayonnement ou contient une plaque (32) constituée en un tel matériau.

9. Installation de radiodiagnostic suivant la revendication 8, caractérisée par le fait que la plaque (32) est intégrée dans un organe de retenue (22) possédant la forme d'un L et dont une branche forme la surface d'application pour le film (6) et dont l'autre branche est réalisée de manière à permettre sa fixation détachable sur la barre de support (21), et que l'organe de support (22), portant le film, du porte-film (7) est monté de façon à pouvoir en être retiré, sur la barre de support (21) pouvant être fixée sur le localisateur.

10. Installation de radiodiagnostic suivant la revendication 9, caractérisée par le fait que la barre de support (21) et son support (17) sur le localisateur (3) sont réalisés avec une section transversale en forme de U ou de V et forment ainsi des mâchoires de serrage (24) ayant une action élastique latérale et qui peuvent être amenées en et hors de prise avec des agrafes (25) de guidage et de retenue, qui s'adaptent à ces mâchoires et sont prévues sur le localisateur.

0 124 714

FIG 1

FIG 2

FIG 3

FIG 4

FIG 7

FIG 5

FIG 6